# EUROPEAN PATENT APPLICATION

(11) **EP 4 275 644 A1**
(43) Date of publication of application: **15.11.2023**
(21) Application number: 22172289.5
(22) Date of filing: 09.05.2022
(51) Int. Cl.: A61B 90/00, A61B 34/30, G06N 3/02, A61B 34/20, A61B 34/00

(54) **SYSTEM AND METHOD FOR ALIGNING MOVEMENT DIRECTION OF INTERVENTIONAL DEVICE IN IMAGE AND CONTROL DIRECTION OF COMMANDS ENTERED BY USER**

(71) Applicant: Koninklijke Philips N.V., 5656 AG Eindhoven (NL)
(72) Inventor: FOTOUHI, Javad, Eindhoven (NL); BALICKI, Marcin A, Eindhoven (NL); SALEHI, Leili, Eindhoven (NL); HAASE, Hans Christian, Eindhoven (NL)
(74) Representative: Philips Intellectual Property & Standards

(57) **Abstract**

A system and method are provided for aligning movement of an interventional device in an image and control commands entered by a user to visualize progress of the interventional device guided by a robot in an anatomical structure. The method incudes receiving a current image of the interventional device in a current position; receiving control input from an input device on a control console for controlling movement of the interventional device; determining a control direction of the input device; estimating a movement direction of the interventional device in the current image based on the control input; estimating a mismatch between the movement direction and the control direction; and adjusting an orientation of the current image relative to the display or an orientation of the input device relative to the control console to align the movement direction of the interventional device and the control direction of the input device of the control console.

## Description

### BACKGROUND

Robotic systems used to perform interventional procedures, such as endovascular procedures, are operated by a user using a control console of a robot controller for steering a bendable interventional device, such as a catheter or a guidewire. The motion and behavior of the interventional devices in a subject (patient) depend on the control inputs that the user provides through the control console, as well as the shape, properties and position of the interventional devices relative to the subject's anatomy. The control inputs may be generated using a control interface of the control console, such as a joystick, where directional movements of the control interface cause corresponding directional movements of the interventional device displayed on a screen.

During an interventional procedure, the user typically steers the interventional device through complicated anatomical structures (e.g., vessels) having high curvatures, bifurcations and tortuous pathways, while viewing the progress in a displayed image, which may be difficult and time consuming. Improper steering may result in multiple failed attempts at performing the interventional procedure, and may lead to damage to the anatomical structure (e.g., hemorrhage), increased procedure time, increased exposure of the subject to imaging radiation, and/or the need to change the interventional device.

Hand-eye coordination of the user is a crucial concern when navigating an interventional instrument under real-time imaging guidance (e.g., fluoroscopy). Facilitating proper hand-eye coordination is particularly challenging when the interventional device is operated remotely from the robot controller, for example, when the control console is away from the subject, e.g., due to radiation safety or other concerns. However, hand-eye coordination suffers when the control direction entered at the control console does not match the movement direction of the interventional device in the displayed image. For example, the interventional device may be shown in the displayed image moving to the left, while the user enters a control input by pushing a joystick on the control console upward. Such misalignments challenge the user's hand-eye coordination when navigating the interventional device, where steering inputs are not intuitive to the movement direction of the interventional device.

### SUMMARY

According to a representative embodiment, a system is provided for aligning movement of an interventional device in an image on a display and control commands entered by a user to visualize progress of the interventional device guided by a robot and configured for insertion into an anatomical structure of a subject. The system includes a display configured to display images of the interventional device in the anatomical structure of the subject; a control console including an input device operable by a user for controlling movement of the interventional device via the robot; at least one processor coupled to the display and the control console; and a non-transitory memory for storing machine executable instructions. When executed by the at least one processor, the instruction cause the at least one processor to receive a current image of the interventional device in the anatomical structure displayed on the display, the current image showing a current position of the interventional device; receive a control input from the control console for controlling a movement of the interventional device from the current position; determine a control direction of the input device relative to the control console based on the control input; estimate a movement direction of the interventional device in the current image on the display based on the control input; estimate a mismatch between the movement direction of the interventional device and the control direction of the input device of the control console; and adjust an orientation of the current image relative to the display or an orientation of the input device relative to the control console to align the movement direction of the interventional device on the display and the control direction of the input device of the control console.

According to another representative embodiment, a system is provided for displaying and controlling the progress of an interventional device configured for insertion into an anatomical structure of a subject, the system comprising:
at least one processor adapted to be coupled to (i) a display and to (ii) a user interface to provide control inputs for controlling movements of the interventional device; and
a non-transitory memory for storing instructions that, when executed by the at least one processor, cause the at least one processor to:
   read a determinate coordinate system associated with the user interface, stored in said memory;
   receive image data of a current image of the interventional device in the anatomical structure displayed or to be displayed on a display, the current image showing a current position of the interventional device;
   receive a control input from the user interface for controlling a movement of the interventional device from the current position, the control input being representative of a control direction in said determinate coordinate system of the user interface;
   estimate from at least the current image data a movement direction of the interventional device based on the control input;
estimate a mismatch between the movement direction of the interventional device and the control direction;
determine a change of orientation of the current image displayed or to be displayed or a change of orientation of the coordinate system of the user interface to align the movement direction of the interventional device in the image and the control direction of the user interface; and
implement the change of orientation, which may be done in connection with a display or a user interface.

The present invention and specification further include the subject-matter of any and all dependent claims 2 to 13.

According to another representative embodiment, a method is provided for aligning movement of an interventional device in an image on a display and control commands entered by a user using a control console to visualize progress of the interventional device guided by a robot and configured for insertion into an anatomical structure of a subject. The method includes receiving a current image of the interventional device in the anatomical structure of the subject, the current image being displayed on the display and showing a current position of the interventional device; receiving a control input from the control console in response to operation by the user of an input device for controlling a movement of the interventional device from the current position via the robot; determining a control direction of the input device relative to the control console based on the control input; estimating a movement direction of the interventional device in the current image on the display based on the control input; estimating a mismatch between the movement direction of the interventional device and the control direction of the input device of the control console; and adjusting an orientation of the current image relative to the display or an orientation of the input device relative to the control console to align the movement direction of the interventional device on the display and the control direction of the input device of the control console.

According to another representative embodiment, a method is provided for displaying and controlling the progress of an interventional device configured for insertion into an anatomical structure of a subject, the method comprising:
receiving a current image data of the interventional device in the anatomical structure of the subject, the current image being displayed or to be displayed on a display and showing a current position of the interventional device;
receiving a control input from a user interface, e.g. in response to operation by a user of an input device, for controlling a movement of the interventional device from the current position, the control input being representative of a control direction in a registered determinate coordinate system of the user interface;
estimating from at least the current image data a movement direction of the interventional device based on the control input;
estimating a mismatch between the movement direction of the interventional device and the control direction;
determining a change of orientation of the current image or a change of orientation of the coordinate system of the user interface to align the movement direction of the interventional device and the control direction of the user interface; and
implementing the change of orientation, which may be done in connection with a display or a user interface.

According to another representative embodiment, it is provided a non-transitory computer readable medium stores instructions, for aligning movement of an interventional device in an image on a display and control commands entered by a user using a control console to visualize progress of the interventional device guided by a robot and configured for insertion into an anatomical structure of a subject. When executed by at least one processor, the instructions cause the at least one processor to receive a current image of the interventional device in the anatomical structure of the subject, the current image being displayed on the display and showing a current position of the interventional device; receive a control input from the control console in response to operation by the user of an input device for controlling a movement of the interventional device from the current position via the robot; determine a control direction of the input device relative to the control console based on the control input; estimate a movement direction of the interventional device in the current image on the display based on the control input; estimate a mismatch between the movement direction of the interventional device and the control direction of the input device of the control console; and adjust an orientation of the current image relative to the display or an orientation of the input device relative to the control console to align the movement direction of the interventional device on the display and the control direction of the input device of the control console.

According to another representative embodiment, it is provided a non-transitory computer readable medium storing instructions, for displaying and controlling the progress of an interventional device configured for insertion into an anatomical structure of a subject, that when executed by at least one processor adapted to be coupled to (i) a display and to (ii) a user interface to provide control inputs for controlling movements of the interventional device, cause the at least one processor to:
read a determinate coordinate system associated with the user interface, stored in said memory;
receive a current image data of the interventional device in the anatomical structure displayed or to be displayed on a display, the current image showing a current position of the interventional device;
receive a control input from the user interface for controlling a movement of the interventional device from the current position, the control input being representative of a control direction in said determinate coordinate system of the user interface;
estimate from at least the current image data a movement direction of the interventional device based on the control input;
estimate a mismatch between the movement direction of the interventional device and the control direction;
determine a change of orientation of the current image or a change of orientation of the coordinate system of the user interface to align the movement direction of the interventional device in the image and the control direction of the user interface; and
implementing the change of orientation, which may be done in connection with a display or a user interface.

### BRIEF DESCRIPTION OF THE DRAWINGS

The example embodiments are best understood from the following detailed description when read with the accompanying drawing figures. It is emphasized that the various features are not necessarily drawn to scale. In fact, the dimensions may be arbitrarily increased or decreased for clarity of discussion. Wherever applicable and practical, like reference numerals refer to like elements.
FIG. 1A shows an illustrative control console for controlling movement of an interventional device in an illustrative displayed image with mismatched control and movement directions.
FIG. 1B shows the illustrative control console and the illustrative displayed image of the interventional device with matching control and movement directions, where orientation of the image is adjusted, according to a representative embodiment.
FIG. 1C shows the illustrative control console and the illustrative displayed image of the interventional device with matching control and movement directions, where orientation of an input device on the control console is adjusted, according to a representative embodiment.
FIG. 2 is a simplified block diagram of a system for aligning movement of an interventional device in a displayed image and directional control commands entered by a user through a control console to visualize progress of the interventional device in an anatomical structure of a subject, according to a representative embodiment.
FIG. 3 is a flow diagram showing a method of aligning movement of an interventional device in a displayed image and directional control commands entered by a user through a control console to visualize progress of the interventional device in an anatomical structure of a subject, according to a representative embodiment.
FIG. 4 shows illustrative displayed images where the movement direction of an interventional device in each image has been estimated by inferring the shape and location of the interventional device and surrounding anatomy of the anatomical structure from the current image and recent past images of the subject, according to a representative embodiment.
FIG. 5 shows illustrative displayed images where the movement direction of an interventional device in a current image has been estimated by applying the first neural network model to obtain future motion vectors for predicting corresponding next images indicative of a future direction of the movement of the interventional device, according to a representative embodiment.
FIG. 6A shows an illustrative control console for controlling directional movement of an interventional device and an illustrative displayed image of the interventional device viewed by a user straight ahead, according to a representative embodiment.
FIG. 6B shows the illustrative control console for controlling directional movement of the interventional device and the illustrative displayed image of the interventional device viewed by the user at an offset angle, with matching directional orientations, according to a representative embodiment.

### DETAILED DESCRIPTION

In the following detailed description, for the purposes of explanation and not limitation, representative embodiments disclosing specific details are set forth in order to provide a thorough understanding of an embodiment according to the present teachings. Descriptions of known systems, devices, materials, methods of operation and methods of manufacture may be omitted so as to avoid obscuring the description of the representative embodiments. Nonetheless, systems, devices, materials and methods that are within the purview of one of ordinary skill in the art are within the scope of the present teachings and may be used in accordance with the representative embodiments. It is to be understood that the terminology used herein is for purposes of describing particular embodiments only and is not intended to be limiting. The defined terms are in addition to the technical and scientific meanings of the defined terms as commonly understood and accepted in the technical field of the present teachings.

It will be understood that, although the terms first, second, third, etc. may be used herein to describe various elements or components, these elements or components should not be limited by these terms. These terms are only used to distinguish one element or component from another element or component. Thus, a first element or component discussed below could be termed a second element or component without departing from the teachings of the inventive concept.

The terminology used herein is for purposes of describing particular embodiments only and is not intended to be limiting. As used in the specification and appended claims, the singular forms of terms "a," "an" and "the" are intended to include both singular and plural forms, unless the context clearly dictates otherwise. Additionally, the terms "comprises," "comprising," and/or similar terms specify the presence of stated features, elements, and/or components, but do not preclude the presence or addition of one or more other features, elements, components, and/or groups thereof. As used herein, the term "and/or" includes any and all combinations of one or more of the associated listed items.

Unless otherwise noted, when an element or component is said to be "connected to," "coupled to," or "adjacent to" another element or component, it will be understood that the element or component can be directly connected or coupled to the other element or component, or intervening elements or components may be present. That is, these and similar terms encompass cases where one or more intermediate elements or components may be employed to connect two elements or components. However, when an element or component is said to be "directly connected" to another element or component, this encompasses only cases where the two elements or components are connected to each other without any intermediate or intervening elements or components.

The present disclosure, through one or more of its various aspects, embodiments and/or specific features or sub-components, is thus intended to bring out one or more of the advantages as specifically noted below. For purposes of explanation and not limitation, example embodiments disclosing specific details are set forth in order to provide a thorough understanding of an embodiment according to the present teachings. However, other embodiments consistent with the present disclosure that depart from specific details disclosed herein remain within the scope of the appended claims. Moreover, descriptions of well-known apparatuses and methods may be omitted so as to not obscure the description of the example embodiments. Such methods and apparatuses are within the scope of the present disclosure.

Generally, the various embodiments described herein provide a system and method that enable a user (interventionalist) of a robotic system to align control direction of an input device operated on a control console of a robot controller with movement direction of an interventional device guided by a robot and visualized in a surgical display during an interventional procedure (e.g., catheterization). To achieve this alignment, either the image on the display is reoriented to match the orientation of the commands entered through the control console, or the directionality of the commands entered through the control console is reoriented to match the visualization. Self-supervised neural network learning, for example, may be employed to increase stability and to enhance the user experience when rotating the displayed images by learning to set visualization parameters based on the forthcoming events in the procedure. Aligning the control direction associated with control commands and associated with movement direction of the interventional device provides visualization results that accommodate more natural hand-eye coordination of the user, which improves manipulation of the interventional device inside the anatomical structure (e.g., vasculature) of the subject under fluoroscopic guidance.

For example, optimal view orientation may be estimated by combining robot data (e.g., kinematics, encoder data, controller inputs) from the robot guiding the interventional device with past image acquisitions showing the interventional device and the surrounding anatomy, a current image acquisition showing the interventional device and the surrounding anatomy, and a prediction of the future shape and location of the interventional device. Incorporating the future shape and location improves stability of the visualization by allowing the data to be temporally filtered to provide smooth transitions between the displayed images.

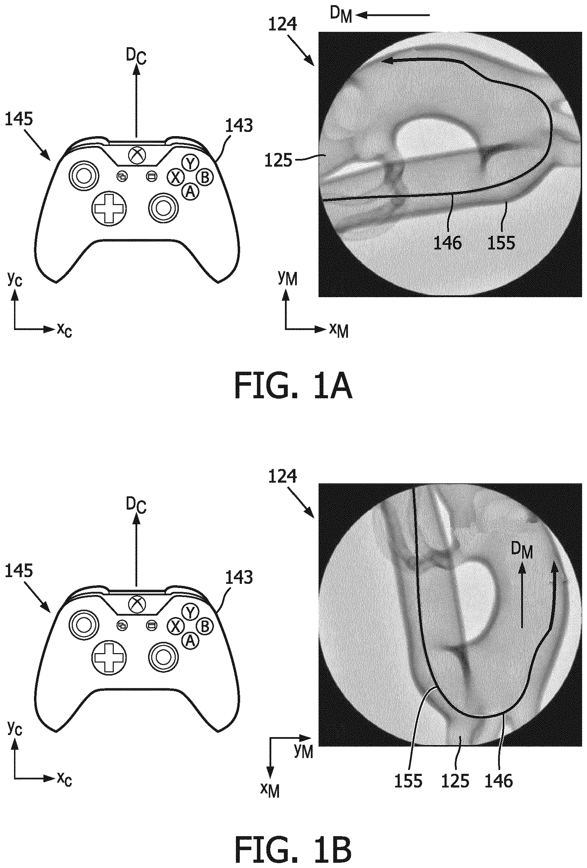

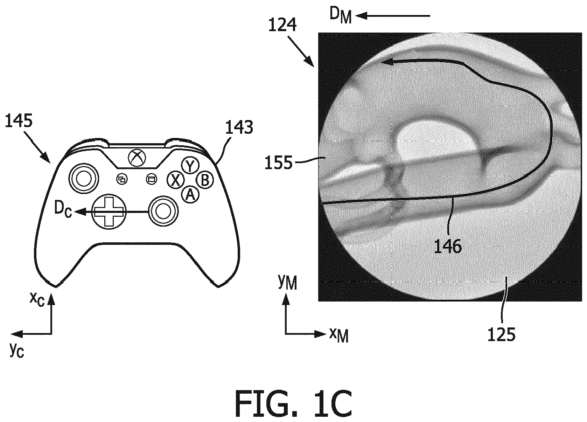

FIG. 1A shows an illustrative control console for controlling movement of an interventional device in an illustrative displayed image with mismatched control and movement directions, and FIG. 1B shows the illustrative control console and the illustrative displayed image of the interventional device with matching control and movement directions, where orientation of the image is adjusted, according to a representative embodiment. FIG. 1C shows the illustrative control console and the illustrative displayed image of the interventional device with matching control and movement directions, where orientation of an input device on the control console is adjusted, according to a representative embodiment.

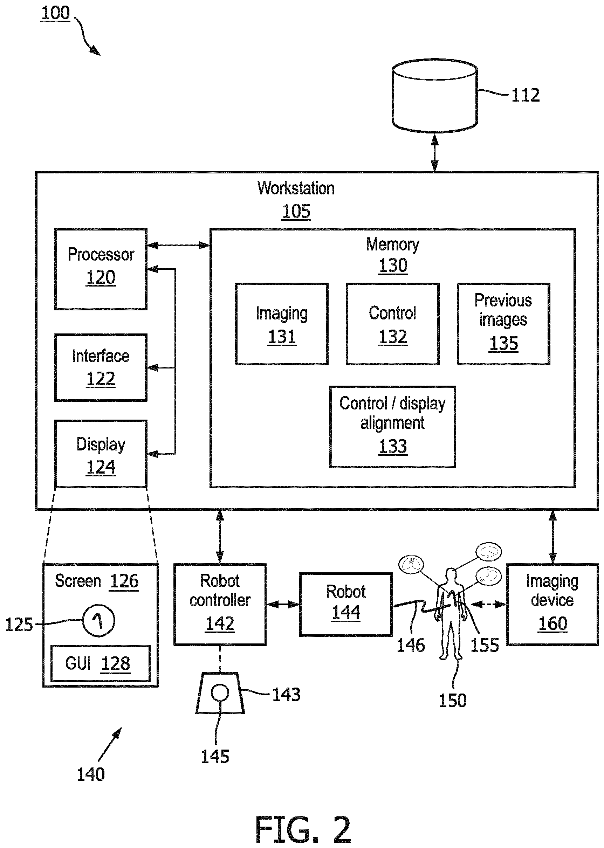

Referring to FIG. 1A, a display 124 shows an image 125 of an interventional device 146 inserted in an anatomical structure 155 of a subject 150, such as a vessel or an artery, for example. The interventional device 146 is guided by a robot under control of a robot controller, such as robot 144 and robot controller 142 discussed below with reference to FIG. 2. The interventional device 146 may be any compatible (non-rigid) medical instrument capable of being controlled robotically, such as a catheter, a guidewire, a stent, a balloon, a sheath, an endoscope, a camera, or a surgical tool, for example.

A control console 143 is configured to interface with the robot controller 142 in order to control movement of the interventional device 146. The control console 143 includes an input device 145 that is operable by the user to control directional movement of the interventional device 146 by applying input controls to the input device 145. The input device 145 may be any compatible interface mechanism operable to indicate the direction of movement of the interventional device 146, such as a joy stick, a thumb stick or a directional pad, for example. The input device 145 may also be configured to control speed of movement of the interventional device 146.

The display 124 may be continuously updated as additional images are acquired, and/or as the user changes the control inputs. The image 125 may be a live fluoroscopic image, for example, although any other type of images acquired in real-time or near real-time, such as ultrasound images, X-ray images, computerized tomography (CT) images, cone-beam CT images, magnetic resonance (MR) images, and positron emission tomography (PET) images, may be incorporated without departing from the scope of the present teachings.

It is understood that the control console 143 may be any type of console capable of interfacing with the robot controller, including consoles that may be specifically designed for interaction with a particular robot controller or off-the-shelf consoles that may be programmed for interaction with a particular robot controller, as would be apparent to one skilled in the art. For example, the control console 143 in the depicted implementation is a specially programmed handheld Xbox^{®} Wireless Controller available from Microsoft^{®} Corporation, and the input device 145 is the left stick on the Xbox^{®} Wireless Controller. For example, the directional orientation of the input device 145 may be changed by reprogramming the application programming interface (API) of the input device 145. It is further understood that the input device 145 may be implemented as a single mechanism (e.g., one that controls both direction and speed), or as multiple mechanisms (e.g., one to control direction and one to control speed) that operate in coordination with one another, without departing from the scope of the present teachings.

A control direction D_{C} of the input device 145 is shown as an arrow relative to the control console 143. The control direction D_{C} is the direction of the control input applied to the input device 145 by the user. For purposes of illustration, the control direction D_{C} may be defined in accordance with imaginary control axes x_{C}, y_{C}. In the depicted example, the control direction D_{C} is shown pointing straight up (in the +y_{C} direction), indicating that the input device 145 is being pushed upward by the user in order to maneuver the interventional device 146 straight ahead (forward).

A movement direction D_{M} of the interventional device 146 is shown as an arrow at the distal end of the interventional device 146 relative to the display 124. The movement direction D_{M} of the interventional device 146 is responsive to the operation (control input) of the input device 145. For purposes of illustration, the movement direction D_{M} may be defined in accordance with imaginary movement axes x_{M}, y_{M}. In the depicted example, the movement direction D_{M} is shown pointing to the left (in the -x_{M} direction), meaning that the distal end of the interventional device 146 moves straight ahead (forward) by moving to the left relative to the display 124 in response to the input device 145 being moved upward relative to the control console 143. This may cause some confusion for the user, who intuitively would be tempted to move the input device 145 to the left to cause the interventional device 146 to move forward in the present orientation of the image 125. However, this control input would actually cause the interventional device 146 to turn left, moving downward relative to the display 124.

In comparison, FIG. 1B shows the control console 143 and the display 124 after the image 125 has been reoriented (e.g., rotated) on the display 124, such that the movement direction D_{M} of the interventional device 146 visually matches (aligns with) the control direction D_{C} of the input device 145, in accordance with a representative embodiment. That is, in the depicted example, the control direction D_{C} is shown pointing straight up, still indicating that the input device 145 is being pushed upward by the user in order to maneuver the interventional device 146 forward. However, the movement direction D_{M} is now shown as an arrow also pointing also pointing straight up, in alignment with the control direction D_{C}. For purposes of illustration, this reorientation of the movement direction D_{M} is indicated by the movement axes x_{M}, y_{M} being rotated clockwise within the display 124. Therefore, the movement direction D_{M} shows the distal end of the interventional device 146 moving forward in the image 125 by moving upward relative to the display 124 in response to the input device 145 being moved upward relative to the control console 143. Accordingly, the user is able to intuitively move the input device 145 in the same direction as the movement of the interventional device 146 in order to intuitively control the interventional device 146 to move forward.

In an alternative embodiment, referring to FIG. 1C, the orientation of the image 125 and the movement direction D_{M} of the interventional device 146 remains unchanged, while the control input of the input device 145 is reoriented, such that the control direction D_{C} matches the movement direction D_{M}. That is, the orientation of the image 125 as shown in FIG. 1A remains the same, meaning that the movement direction D_{M} of the interventional device 146 is to the left when it is moved forward. Meanwhile, the orientation of the input device 145 is changed relative to the control console 143, so that moving the input device 145 to the left, such that the control direction D_{C} points to the left, causes the interventional device 146 to move forward in the image 125.

That is, FIG. 1C shows the control console 143 and the display 124 after the input device 145 has been reoriented (e.g., rotated) relative to the control console 143, such that the control direction D_{C} of the input device 145 visually matches (aligns with) the movement direction D_{M} of the interventional device 146 in the image 125. In the depicted example, the movement direction D_{M} is shown as an arrow pointing to the left, still indicating that the interventional device 146 is still moving to the left in the image 125 when being controlled to move forward. The control direction D_{C} is also shown pointing to the left, indicating that the input device 145 has been reoriented such that the user pushes the input device 145 to the left in order to maneuver the interventional device 146 forward. For purposes of illustration, this reorientation of the control direction D_{C} is indicated by the control axes x_{C}, y_{C} being rotated counterclockwise relative to the control console 143. Accordingly, the user is able to intuitively move the input device 145 in the same direction as the movement of the interventional device 146 in order to control the interventional device 146 to move forward.

In various embodiments, the interventional device 146 may be a coaxial device, including an inner device and a surrounding outer device. For example, the interventional device 146 may comprise a guidewire inserted through a catheter, each of which is separately controllable. In this case, the user may select one of the inner device and the outer device to control determination of the orientation of the image 125 on the display 124 for alignment of the control direction D_{C} and the movement direction D_{M}. That is, the user may select the most distal one of the inner and outer devices as the reference for determining the alignment, the most proximal one of the inner and outer devices as the reference for determining the alignment, or an average direction of the inner and outer devices for determining the alignment.

Other reference metrics of the interventional device 146 (whether coaxial or not) for estimating the desired orientation alignment include considering the shape and/or direction of the interventional device 146 for a predetermined section of N millimeters and/or pixels, a section of the interventional device 146 that is actively steerable, or a distal section of the interventional device that is straight, for example. When the interventional device 146 is an articulated device, the orientation alignment may be determined based on the most distal segment of the interventional device, the most proximal segment of the interventional device, the average of N distal segments, or the average of N proximal segments (where N is a positive integer greater than 1), for example.

FIG. 2 is a simplified block diagram of a system for aligning movement of an interventional device in a displayed image and directional control commands entered by a user through a control console to visualize progress of the interventional device in an anatomical structure of a subject, according to a representative embodiment.

Referring to FIG. 2, system 100 includes a workstation 105 for implementing and/or managing the processes described herein with regard to aligning movement of the interventional device 146 in the image 125 on the display 124 and control commands entered by a user to visualize progress of the interventional device 146 in the anatomical structure 155 of the subject (patient) 150. The workstation 105 includes one or more processors indicated by processor 120, one or more memories indicated by memory 130, a user interface 122 and the display 124. The processor 120 interfaces with a robotic system 140 through a control module 132, where the robotic system 140 includes the robot controller 142, the control console 143 and the robot 144. The robot controller 142 is configured to control movement of the robot 144 in response to user control inputs received through operation of the input device 145 of the control console 143. The robot 144 is attached to or integrated with the interventional device 146. The robot 144 may include segments, joints, servo motors and other control features operable for moving and positioning the interventional device 146 in multiple degrees of freedom (DOFs) in response to control signals received from the robot controller 142. In the depicted embodiment, the robot controller 142 is shown separately from processor 120 in the workstation 105 for purposes of illustration. It is understood, however, that all or part of the functionality of the robot controller 142 may be incorporated into the processor 120, or vice versa, without departing from the scope of the present teachings.

The user interfaces with the robot controller 142 using the control console 143. The control console 143 may be a handheld control console, such as a specially programmed Xbox^{®} Wireless Controller available from Microsoft^{®} Corporation, discussed above, although any type of compatible control console may be incorporated without departing from the scope of the present teachings. The control console 143 may communicate with the robot controller 142 via a wireless connection, indicated by a dashed line, such as Bluetooth (IEEE 802.15.1), ZigBee (IEEE 802.15.4) or WiFi (IEEE 802.11), for example, either directly or through local or wide area networks. Alternatively, the control console 143 may communicate with the robot controller 142 via a wired connection, such as transmission lines, cables, coaxial cables, or fiber-optic cables, for example.

The processor 120 also interfaces with an imaging device 160 through an imaging module 131. The imaging device 160 may be any of various types of medical imaging device/modality, including a fixed or mobile C-arm fluoroscopy system, an X-ray imaging device, a CT scan device, an MR imaging device, a PET scan device, or an ultrasound imaging device, for example. The imaging device 160 may include single or multiple imaging modalities.

The memory 130 stores instructions executable by the processor 120. When executed, the instructions cause the processor 120 to implement one or more processes for aligning movement of the interventional device 146 in the image 125 and control commands entered by the user through the control console 143 to intuitively visualize progress of the interventional device 146 in the anatomical structure 155. For purposes of illustration, the memory 130 is shown to include software modules, each of which includes the instructions corresponding to an associated capability of the system 100, discussed below.

The processor 120 is representative of one or more processing devices, and may be implemented by field programmable gate arrays (FPGAs), application specific integrated circuits (ASICs), a digital signal processor (DSP), a general purpose computer, a central processing unit, a graphical processing unit, a computer processor, a microprocessor, a microcontroller, a state machine, programmable logic device, or combinations thereof, using any combination of hardware, software, firmware, hard-wired logic circuits, or combinations thereof. Any processing unit or processor herein may include multiple processors, parallel processors, or both. Multiple processors may be included in, or coupled to, a single device or multiple devices. The term "processor" as used herein encompasses an electronic component able to execute a program or machine executable instruction. A processor may also refer to a collection of processors within a single computer system or distributed among multiple computer systems, such as in a cloud-based or other multi-site application. Programs have software instructions performed by one or multiple processors that may be within the same computing device or which may be distributed across multiple computing devices.

The memory 130 may include main memory and/or static memory, where such memories may communicate with each other and the processor 120 via one or more buses. The memory 130 may be implemented by any number, type and combination of random-access memory (RAM) and read-only memory (ROM), for example, and may store various types of information, such as software algorithms, artificial intelligence (AI) machine learning models, and computer programs, all of which are executable by the processor 120. The various types of ROM and RAM may include any number, type and combination of computer readable storage media, such as a disk drive, flash memory, an electrically programmable read-only memory (EPROM), an electrically erasable and programmable read only memory (EEPROM), registers, a hard disk, a removable disk, tape, compact disk read only memory (CD-ROM), digital versatile disk (DVD), floppy disk, Blu-ray disk, a universal serial bus (USB) drive, or any other form of storage medium known in the art. The memory 130 is a tangible storage medium for storing data and executable software instructions, and is non-transitory during the time software instructions are stored therein. As used herein, the term "non-transitory" is to be interpreted not as an eternal characteristic of a state, but as a characteristic of a state that will last for a period. The term "non-transitory" specifically disavows fleeting characteristics such as characteristics of a carrier wave or signal or other forms that exist only transitorily in any place at any time. The memory 130 may store software instructions and/or computer readable code that enable performance of various functions. The memory 130 may be secure and/or encrypted, or unsecure and/or unencrypted.

The system 100 may also include a database 112 for storing information that may be used by the various software modules of the memory 130. For example, the database 112 may include image data from previously obtained images of the subject 150 and/or of other similarly situated subjects having the same or similar interventional procedures as the subject 150, together with control input data indicating the control input to a robot corresponding to the previously obtained images. The stored image data and corresponding control input data may be used for training AI machine learning models, such as neural network models, for example, as discussed below. The database 112 may be implemented by any number, type and combination of RAM and ROM, for example. The various types of ROM and RAM may include any number, type and combination of computer readable storage media, such as a disk drive, flash memory, EPROM, EEPROM, registers, a hard disk, a removable disk, tape, CD-ROM, DVD, floppy disk, Blu-ray disk, USB drive, or any other form of storage medium known in the art. The database 112 comprises tangible storage mediums for storing data and executable software instructions and is non-transitory during the time data and software instructions are stored therein. The database 112 may be secure and/or encrypted, or unsecure and/or unencrypted. For purposes of illustration, the database 112 is shown as a separate storage medium, although it is understood that it may be combined with and/or included in the memory 130, without departing from the scope of the present teachings.

The processor 120 may include or have access to an AI engine, which may be implemented as software that provides artificial intelligence (e.g., neural network models) and applies machine learning described herein. The AI engine may reside in any of various components in addition to or other than the processor 120, such as the memory 130, an external server, and/or the cloud, for example. When the AI engine is implemented in a cloud, such as at a data center, for example, the AI engine may be connected to the processor 120 via the internet using one or more wired and/or wireless connection(s).

The user interface 122 is configured to provide information and data output by the processor 120, the memory 130 and/or the robot controller 142 to the user, and/or to receive information and data input by the user. That is, the user interface 122 enables the user to enter data and to control or manipulate aspects of the processes described herein, and also enables the processor 120 to indicate effects of the user's input. All or a portion of the user interface 122 may be implemented by a graphical user interface (GUI), such as GUI 128 viewable on a screen 126, discussed below. The user interface 122 may include one or more interface devices, such as a mouse, a keyboard, a trackball, a joystick, a microphone, a video camera, a touchpad, a touchscreen, voice or gesture recognition captured by a microphone or video camera, for example.

The display 124 may be a monitor such as a computer monitor, a television, a liquid crystal display (LCD), an organic light emitting diode (OLED), a flat panel display, a solid-state display, or a cathode ray tube (CRT) display, or an electronic whiteboard, for example. The display 124 includes the screen 126 for viewing the images of the subject 150 (e.g., the image 125), along with various features described herein to assist the user in accurately and efficiently reading the images, as well as the GUI 128 to enable the user to interact with the displayed images and features. The user is able to personalize the various features of the GUI 128, discussed below, by creating specific alerts and reminders, for example.

Referring to the memory 130, the various modules store sets of data and instructions executable by the processor 120 to align displayed movement of the interventional device 146 in the image 125 and directions of control commands entered by the user through the control console 143 to intuitively visualize progress of the interventional device 146 in the anatomical structure 155.

Imaging module 131 is configured to receive and process images of the anatomical structure 155 in the subject 150 and the interventional device 146, including a series of current images 125 being viewed by the user during the interventional procedure. Each image 125 may be received in real time from the imaging device 160 during a contemporaneous current imaging session of the subject 150. Alternatively, the image 125 may be an image of the subject 150 previously acquired in the current imaging session, where the imaging has been paused, for example, to reduce exposure of the subject 150 to radiation. Likewise, the image 125 may be retrieved from the database 112, which stores images obtained during previous imaging session(s) or earlier in the current imaging session (from single or multiple imaging modalities). The current image 125 is displayed on the screen 126 to enable analysis by the user and navigation of the interventional device 146 via the robot 144.

Control module 132 is configured to receive control inputs from the user via the control console 143 and the robot controller 142 for controlling the robot 144 to guide movement of the interventional device 146, and to determine control directions of the input device 145 relative to the control console 143 based on the control inputs. The control inputs include maneuvering instructions, such as articulation, rotation, translation, distance, velocity, and acceleration of the robot 144, for example, for moving the interventional device 146 in the anatomical structure 155. The control directions are the directions in which the user operates the input device 145 on the control console 143 in order to control movement of the interventional device 146. For example, the control directions may be determined relative to the control axes x_{C}, y_{C} of the control console 143, as shown in FIGs. 1A and 1B. In this case, the control directions may be indicated as vectors extending from the origin to x, y coordinates along the control axes x_{C}, y_{C}, where the length of the vector may be determined by the amount of time the user holds the input device in the desired control direction, for example. The control directions of the input device 145 may be reoriented by the control module 132 in relation to the control console 143, which may be referred to as reorienting the input device. For example, operating the input device 145 straight ahead (upward) may be reoriented from causing the interventional device 146 to move forward to causing the interventional device 146 to move to the left. In this manner, the control direction D_{C} of the input device 145 may be aligned with the movement direction D_{M} of the interventional device shown on the display 124.

In an embodiment, the control inputs may be initially untriggered, in that the user enters the control inputs without them being executed by the robot controller 142. In this manner, the processor 120 is able to predict the effect of the control inputs on the trajectory of the interventional device 146 prior to the robot controller 142 controlling the robot 144 to actually move the interventional device 146 in response to the control inputs. This enables the user to determine whether the untriggered control inputs are appropriate before action is taken to implement the control inputs.

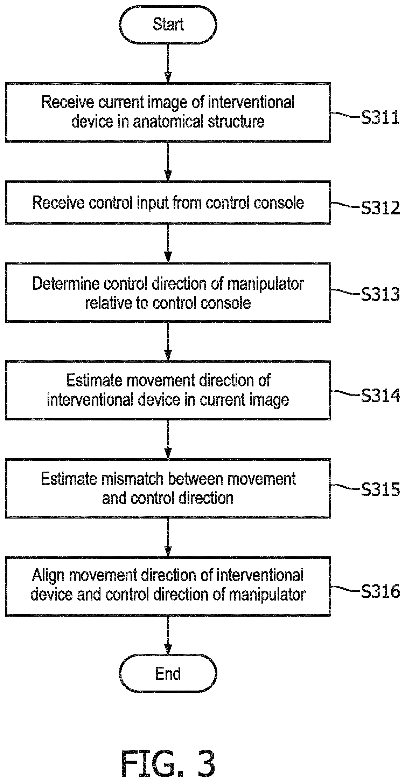

The control/display alignment module 133 is configured to align movement of the interventional device 146 in the current image 125 on the display 124 and the control inputs provided by the control module 132 in order to enable the user to visualize progress of the interventional device 146 while intuitively entering the control inputs. In an embodiment, as discussed below in more detail with reference to FIG. 3, the control/display alignment module 133 estimates movement directions of the interventional device 146 in the current image 125 based on the control inputs. The estimated movement direction is the direction in which the interventional device 146 will most likely move in response to the control input by the user by operating the input device 145 on the control console 143. Generally, the estimated movement direction is determined from control input provided by the control module 132 indicating the control direction of the input device 145 and image data of the current image 125 provided by the imaging module 131. For example, the movement direction may be estimated relative to the movement axes x_{M}, y_{M} of the current image 125, as shown in FIGs. 1A and 1B. In this case, the movement direction may be indicated as a vector extending from the origin to x, y coordinates along the movement axes x_{M}, y_{M}.

The control/display alignment module 133 estimates a mismatch between the movement direction of the interventional device 146 and the control direction applied to the input device 145 of the control console 143. The control/display alignment module 133 then aligns the movement direction of the interventional device 146 on the display 124 and the control direction of the input device 145 on the control console 143 by compensating for the estimated mismatch, as discussed below. The movement and control directions may be aligned either by adjusting the orientation of the current image 125 on the display 124 so that the movement direction of the interventional device 146 aligns with the control direction of the input device 145, or by adjusting the orientation of the input device 145 with respect to the control console 143 so that the control direction aligns with the movement direction of the interventional device 146 in the current image 125 on the display 124. The control/display alignment module 133 may include neural network models for estimating the movement directions of the interventional device and/or for estimating the mismatch between the movement direction of the interventional device and the control directions of the control inputs provide by the input device 145, as discussed below.

Previous image module 135 is configured to receive previous image data from previous images, including previous images of the subject 150, for example, from the imaging device 160 and/or the database 112. The previous images include images of the subject 150 acquired earlier in the current imaging session and/or images of the subject 150 acquired during previous imaging sessions involving the same type of interventional procedure. The images may have been acquired from a single or multiple imaging modalities. In an embodiment, the previous image module 135 may also receive previous images of other similarly situated subjects having the same or similar interventional procedures as the subject 150 having the same type of interventional procedure. The previous image data of the subject 150 and/or of other subjects may include control inputs corresponding to the previous images, indicating the commands entered by the user to place at the time the previous image was acquired. The previous images of the subject 150 and/or of other subjects may be used for training neural network models, for example, as discussed below.

FIG. 3 is a flow diagram of a method of aligning movement of an interventional device in a displayed image and directional control commands entered by a user through a control console to visualize progress of the interventional device in an anatomical structure of a subject, according to a representative embodiment. The method may be implemented by the system 100, discussed above, under control of the processor 120 executing instructions stored as the various software modules in the memory 130, for example.

Referring to FIG. 3, the method includes receiving a current image (e.g., current image 125) in block S311 showing an interventional device (e.g., interventional device 146) in the anatomical structure (e.g., anatomical structure 155), where the current image is displayed on a display (e.g., display 124). The current image shows a current position of the interventional device within the anatomical structure. The process described below assumes the current image is a two-dimensional image, although it may apply to three-dimensional images or three-dimensional fluoroscopy images (four-dimensional images), without departing from the scope of the present teachings. Three-dimensional and four-dimensional image data may be displayed by projecting them into a two-dimensional display, e.g., as digitally-reconstructed-radiographs (DRRs), or by displaying a two-dimensional slice through the three-dimensional image data, e.g., in axial or sagittal planes. Using three-dimensional or four-dimensional image data, the display of the projection images or image slices may be aligned with the robot controller in accordance with the processes described herein.

In block S312, a control input is received from a control console (e.g., control console 143) for controlling a movement of the interventional device from the current position. The control input may be initiated by the user at the control console by operation of an input device (e.g., input device 145), and provided to the robot controller for controlling the robot to move the interventional device. The control input has a corresponding control direction (D_{C}), which is the direction in which an input device on the control console is moved relative to the control console in order to affect a corresponding movement of the interventional device. For example, when the input device is a joystick, the input device may be pushed upward to cause a forward movement, downward to cause a backward movement, left to cause a left movement, and right to cause a right movement of the interventional device.

In block S313, the control direction of the input device is determined relative to the control console based on the control input. The control direction is the direction in which the user operates the input device on the control console in order to control movement of the interventional device. The control direction may be determined relative to the control axes x_{C}, y_{C} of the control console 143, for example, as shown in FIGs. 1A and 1B, as discussed above.

In block S314, a movement direction (D_{M}) of the interventional device in the current image on the display is estimated based on the control input. The estimated movement direction is the direction in which the interventional device will most likely move in response to the control input by the user by maneuvering the input device on the control console. Generally, the estimated movement direction is determined from control data indicating the control direction of the input device and image data from the current image showing the interventional device within the anatomical structure. For example, the movement direction may be estimated relative to the movement axes x_{M}, y_{M} of the image 125, as shown in FIGs. 1A and 1B, discussed above.

In an embodiment, estimating the movement direction of the interventional device includes inferring a shape and location of the interventional device and surrounding anatomy of the anatomical structure from the current image and recent past images of the subject. The interventional device and the anatomical structure may be identified in the images using any compatible image recognition techniques, such as edge detection, for example. The image data showing the anatomical structure will generally indicate a lumen defined by the anatomical structure in which the interventional device is located, where the walls of the lumen may be identifiable using the edge detection. Likewise, the edge detection will indicate the shape and location of the interventional device within the walls of the lumen, where a longitudinal axis of the interventional device is generally aligned with a longitudinal axis of the anatomical structure. The movement direction of the interventional device may then be estimated based on the shapes and locations of the interventional device and the surrounding anatomy of the anatomical structure within consecutive recent past images. For example, the consecutive past images may show the interventional device advancing in the lumen of the anatomical structure along the longitudinal axis. The estimated movement direction is therefore effectively a projection of the direction established by the shapes and locations of the interventional device provided by the consecutive recent past images. The device detection may be achieved by an image segmentation or object detection neural network model that receives current and/or past images, and identifies pixels within each image that are occupied by the interventional device and/or the anatomical structure, as is known in the art.

In another embodiment, estimating the movement direction of the interventional device includes establishing a current motion vector for the interventional device using recent past images and corresponding control inputs. The current motion vector represents a direction and a magnitude of displacement of the interventional device moving through the anatomical structure as estimated from the recent past images. For example, the current motion vector may be established by locating a predetermined point on the interventional device, such as the distal tip or the location of a marker, for example, in each of the recent past images and effectively connecting these locations over a predetermined number of recent past images. In an embodiment, the current motion vector may be estimated by applying a first neural network model to the current image and recent past images. The first neural network model is discussed below in more detail. Again, the interventional device and the anatomical structure, as well as the locations of the predetermined points, may be identified using any compatible shape recognition techniques, such as edge detection, for example.

A future motion vector is then predicted for corresponding next images indicative of a future direction of the movement of the interventional device based on the current motion vector established from the recent past images. The future motion vector may be predicted by applying the first neural network model to the current motion vector based on the recent past images. In an embodiment, the first neural network model may also receive robotics data along with the recent past images for predicting the future motion vector. The robotics data indicates positions and orientations of the robot while controlling the medical instrument in corresponding images, and may include kinematics data, joint information, encoder information, velocities, accelerations, end-effector position, force, torque, and/or ranges and limits, for example. The first neural network model may be self-supervised, for example, in which case training the first neural network model may be performed without any explicit labelled data or annotations. According to the first neural network model, a direction of the future motion vector indicates a predicted direction of movement and a length of the future motion vector indicates a number of future frames of the next images are needed for the movement of the interventional device to be fully realized. Generally, the first neural network model compares the current motion vector to motion vectors determined with regard to training images, discussed below, in which similar current motion vectors were provided in similar scenarios with regard to the type of anatomical structure and corresponding control inputs used to position the interventional device as shown in the training images. The first neural network model may include recurrent convolutional layers or transformer architectures, for example. The movement direction of the interventional device is then estimated based on the predicted future motion vector.

The first neural network model may be initially trained using training data from training images that include motion vectors and corresponding control inputs associated with the training images (e.g., from previous images module 135). The training images may be previous images of the same interventional device and corresponding control inputs to the robot for guiding movement of the interventional device through the anatomical structure of the same subject, as shown in the previous images. In addition, or alternatively, the training images may be previous images from other, similar interventional procedures of other subjects using the same or similar interventional devices and corresponding control inputs to the robot for guiding movement of the interventional devices as shown in the previous images.

The first neural network model is provided in two processes, which may generally be referred to as training and inference processes. During the training process, appropriate parameters of the first neural network model are learned based on historical data, which includes an optimization process during which the first neural network parameters will change. The optimization process is iterative, where during each iteration, the first neural network model uses two inputs, including the current images and recent past images from historical cases, and estimates the future motion vector using these two inputs. In an embodiment, the first neural network model may further use current robotics data corresponding to the current images and recent past robotics data corresponding to the recent past images as input. The future motion vector may be represented in various forms, such as key-point coordinates at the start and end of the future motion vector, for example. During the inference process, the trained first neural network model predicts the future motion vectors. The current and recent past images (and robotics data) are forward-passed through the first neural network model during the interventional procedure, and respective future motion vectors are estimated based on the same.

More particularly, training the first neural network model may include capturing spatial context and temporal context of the previous images. The spatial context may be captured using convolutional layers, which consist of a sliding window or kernel representing a matrix of weights that slide over the input images and perform element-wise multiplication with the overlapping part of the input image and summing the results into an output feature map. The temporal context may be captured using temporal connections across layers, such as by using recurrent neural networks (RNNs), long-short term memory (LSTM), transformers, and the like.

The neural network training may be supervised training, self-supervised training, or unsupervised training, for example. In supervised training, labels are explicitly predefined. In self-supervised training, current image and control input acquisitions may serve as outputs (labels) for previous image and corresponding control input acquisitions. That is, subsequent image (and robotics) data may be used as labels for the previous image (and robotics) data points, such that there is no need for explicit annotation. In unsupervised learning, image (and robotics) data are clustered such that different clusters indicate different levels of misalignment. For example, one cluster may be associated with image (and robotics) data with 90-degree misalignment, another cluster may be associated with image (and robotics) data with 180-degree misalignment, and so on. Various architectures may be used for unsupervised training, such as auto-encoders and variational auto-encoders, for example. During the training phase, the first neural network model will learn appropriate representations from retrospective data where previous image frames 1, ..., n-1 are used to predict the movement directions of the interventional device in the following m time points (image frames n, ..., n+m).

The previous images and corresponding control input data may be fed together in the earliest layer of the first neural network model. The previous image data may include fluoroscopic images, or segmentation maps from different devices or anatomies within the image, for example. The previous control input data may include measurements from the control console and/or the robot controller, kinematics of the system with regard to articulation, rotation and translation, as well as velocity and acceleration. Alternatively, the previous control input data may be used at an intermediate or latent layer of the first neural network model, acting as a transformation applied to the representation learned from the previous image frames 1, ..., n-1. In this case, the control input data may also be passed through a series of fully connected layers before merging with the convolutional network. The predicted output in the future image frames n, ..., n+m will produce different trajectories having different movement directions for different robot transformations or control input data. Another implementation may use two separate neural networks as the first neural network model, one for the imaging data and one for control input data. In this case, the two neural networks will share weights or feature maps at some intermediary layers. The training of the first neural network model is performed iteratively, where at each iteration, a batch of corresponding previous image and corresponding previous control input data are fed into the first neural network model. The training is preceded by minimizing a similarity loss, such as a binary-cross-entropy or intensity loss, as would be apparent to one skilled in the art.

In block S315, a mismatch is estimated between the movement direction of the interventional device and the control direction of the input device. As discussed above, the control direction is the direction in which the input device on the control console is moved by the user relative to the control console to affect a corresponding movement of the interventional device, and the movement direction is the direction in which the interventional device will move relative to the display in response to the movement of the input device in the control direction. The mismatch refers to an angular difference between control direction and the movement direction in a common reference space. In FIGs. 1A and 1B, for example, the mismatch between the movement direction of the interventional device and the control direction of the input device is about 90 degrees, meaning that movement of the input device straight up (e.g., 90 degrees relative to the control axes x_{C}, y_{C}) causes movement of the interventional device to the left (e.g., 180 degrees relative to the movement axes x_{M}, y_{M}).

In an embodiment, the mismatch may be estimated by applying a second neural network model to the movement direction of the interventional device and the control direction of the input device. Like the first neural network model, the second neural network model may include supervised, self-supervised, or unsupervised training. In an embodiment, the second neural network model directly outputs a single predicted value that represents a rotation that would restore alignment between the movement of the interventional device in the displayed image and the control direction of the directional control commands entered by the user through the control console. The final layer of the second neural network model may include a function that would normalize the predictions within a finite range (e.g., 0 to 1, or -1 to 1), such as a softmax function, a sigmoid function, or a hyperbolic tangent (tanh) function, for example. The output may then rescale between 0 and 360, indicating the desired rotation.

The second neural network model may be initially trained using training data from training images that show movement directions of interventional devices and corresponding control directions applied to the input device of the control console, in substantially the same manner as discussed above with regard to the first neural network model. The training images may be previous images of the same interventional device and corresponding control inputs to the robot for guiding movement of the interventional device through the anatomical structure of the same subject, as shown in the previous images. In addition, or alternatively, the training images may be previous images from other, similar interventional procedures of other subjects using the same or similar interventional devices and corresponding control inputs to the robot for guiding movement of the interventional devices as shown in the previous images. In an embodiment, the second neural network model may also receive robotics data along with the training images for estimating the mismatch between the movement direction of the interventional device and the control direction of the input device. The robotics data indicates positions and orientations of the robot while controlling the medical instrument in corresponding training images, and may include kinematics data, joint information, encoder information, velocities, accelerations, end-effector position, force, torque, and/or ranges and limits, for example, as discussed above.

In block S316, movement direction of the interventional device on the display and control direction of the input device are aligned (matched) by compensating for the mismatch estimated in block S315. The movement and control directions may be aligned either by adjusting the orientation of the current image on the display so that the movement direction of the interventional device aligns with the control direction of the input device, or by adjusting the orientation of the input device with respect to the control console so that the control direction aligns with the movement direction of the interventional device in the current image on the display. Aligning the movement and control directions provides more intuitive control by the user and improves hand-eye coordination, which in return simplify the interaction with the system, minimize the chance of perforation or damage to tissue during the interventional procedure, reduces procedure time, and reduces radiation exposure (when x-ray imaging is involved).

To align the movement and control directions, the current image may be reoriented with respect to the display, e.g., by rotating the movement axes x_{M}, y_{M}, to provide an optimal view orientation of the interventional device's motion, such that the directional movement of the interventional device on the display matches the control movement of the input device on the control console. For example, the current image may be rotated such that the movement direction of the interventional device is in the upward direction to match the upward operation of the input device, as shown in FIG 1B. In an embodiment, the current image may be reoriented by rotating the imaging device (e.g., imaging device 160) itself, used to acquire the current image. When the current image is to be rotated, the processor (e.g., processor 120) may apply a rotation operation to the raw image data and create a new copy of the current comprising the rotated raw image data. The rotated current image is then rendered on the display. Digital images are typically stored as a matrix of elements, e.g., pixels, in which case a rotation matrix is applied to the matrix of elements, resulting in the rotated current image.

Alternatively, the input device may be reoriented with respect to the control console e.g., by rotating the control axes x_{C}, y_{C}, to provide an optimal input coordinates to the robot controller, such that the control direction of the input device matches the movement direction of the interventional device on the display. For example, the functionality the control console (e.g., the input device API) may be dynamically reprogrammed, such that the user enters input in the left control direction to match the displayed left movement direction of the interventional device, as shown in FIG. 1C. In this case, processor changes the assignment of physical control elements of the input device on the control console. A single vector of numerical values can define the behavior of each control element. For example, a thumb stick on the control console that moves the interventional device along the x-axis may be defined as vector [1, 0, 0], and a thumb stick moving along the y-axis may be defined as vector [0, 1, 0]. To reorient the control elements, each of the vectors may be rotated via a rotation matrix, resulting in a new vector corresponding to a different direction. The rotated vectors are then transferred from the processor to the robot controller.

In embodiment, the user may be alerted to the reorientation of the current image with respect to the display or the reorientation of the input device with respect to the control console by a haptic or aural indicator, for example. Likewise, the user may be alerted to certain interim stages of the reorientation of the current image with respect to the display or the reorientation of the input device with respect to the control console, such as rotation of the image or the input device every 30 degrees or 45 degrees, for example.

In an embodiment, the interventional device in its original orientation may be displayed simultaneously in a reduced size, e.g., as a picture-in-picture, while the current image is reoriented to compensate for the mismatch. Also, in an embodiment, the current image may be reoriented (e.g., rotated) in a continuous fashion or in increments to align the movement direction of the interventional device with the control direction of the input device. For example, the current images may be reoriented in discrete angular steps, such as 45 degrees or 90 degrees.

The steps shown in FIG. 3 are then repeated during the interventional procedure, thereby providing real-time feedback to the user as the user controls the movement of the interventional device using the control console. That is, subsequent images of the interventional device (which may or may not be next consecutive images) are sequentially displayed as the current image on the display. The movement direction of the interventional device on the display and control direction of the input device on the control console are determined for each of the subsequent images. The movement and control directions are aligned, if needed, by respectively compensating for estimated mismatches between the two. Accordingly, real-time feedback enabling intuitive operation of the interventional device is provided to the user when viewing the display throughout the interventional procedure.

In an embodiment, the alignment of the movement direction of the interventional device in the image and the control direction of the input device on the control console, and compensating for the mismatch between the two, may be based on vessel roadmapping with contrast injection in the anatomical structure. In this case, a digitally subtracted angiogram (DSA) image and a fluoroscopy image are stacked along the channel dimension and will concurrently feed the first neural network model during training and for estimating the movement direction of the interventional device, and the second neural network model during training and for estimating the mismatch between the movement direction of the interventional device and the control direction of the input device.

In an embodiment, a third neural network model may be trained to learn upcoming workflow and interventional device changes. The current image may then be rotated automatically with respect to the display, indicating the next appropriate orientation of the interventional device that should be achieved in the forthcoming steps such that the movement direction matches the control direction. Transitions between orientations of the interventional device may be triggered by the location and/or shape of the interventional device relative to the anatomical structure for a given task. The third neural network model may similarly use pairs of previous image data and corresponding control inputs to learn the optimal rotations in the future. The robot controller will then automatically apply the optimal rotations to the current image in the display.

In an embodiment, a fourth neural network model may be trained to learn articulation of the interventional device by the robot and the relationship to articulation of the input device. The fourth neural network is then able to estimate future shapes of the interventional device in the images by applying data indicating the movements of the input device.

As described above with reference to block S314 of FIG. 3, the movement direction of the interventional device in the current image on the display may be estimated according to various embodiments. The estimated movement direction may then be used in estimating the mismatch between the movement direction of the interventional device and the control direction applied to the input device of the control console (block S315) and aligning the movement direction of the interventional device on the display and the control direction of the input device to compensate for the estimated mismatch (block S316).

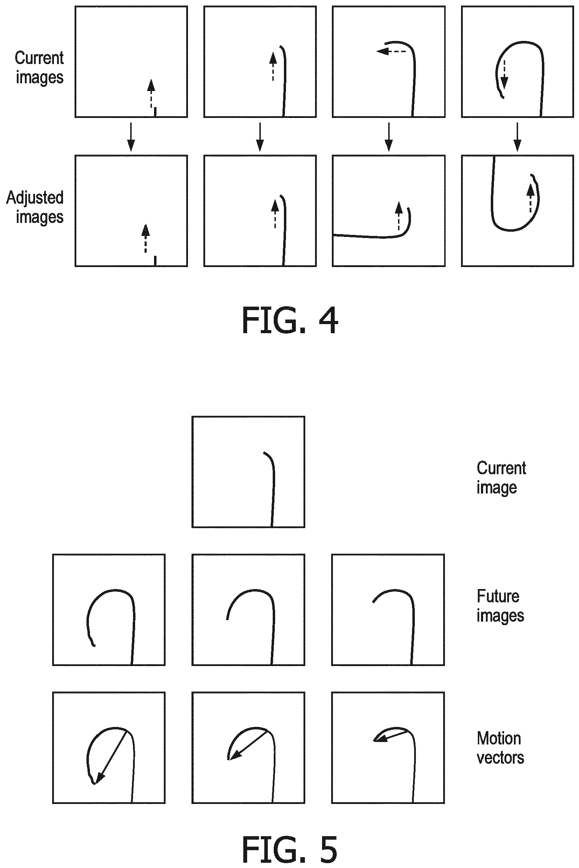

In this context, FIG. 4 shows illustrative displayed images where the movement direction of an interventional device in each image has been estimated by inferring the shape and location of the interventional device and surrounding anatomy of the anatomical structure from the current image and recent past images of the subject, while FIG. 5 shows illustrative displayed images where the movement direction of an interventional device in a current image has been estimated by applying the first neural network model to obtain future motion vectors for predicting corresponding next images indicative of a future direction of the movement of the interventional device, according to representative embodiments. Notably, FIGs. 4 and 5 show mock images of just the interventional devices, for clarity. It is understood, though, that in practice the images would also include the surrounding anatomies of the anatomical structures, which are also used to provide sufficient context to infer the shape and location of the interventional devices and to train the first neural network, as discussed above.

Referring to FIG. 4, the entire shape or the distal segment (or alternatively the proximal segment) of the interventional device may be used to set the optimal orientation. The top row shows four consecutively acquired current images of the interventional device, together with arrows indicating the movement directions of a distal segment of the interventional device at the time the corresponding current image was acquired. The bottom row shows the four consecutively acquired current images of the interventional device following adjustment (e.g., rotation) of the current images to align the movement directions of the interventional device with the corresponding control directions of the input device. As shown, the arrows indicating the movement directions of the distal segment of the interventional device in the bottom row of images are all pointing in the same direction (upward), indicating that the control direction of the input device is also upward relative to the control console, e.g., as shown in FIGs. 1A and 1B.

Referring to FIG. 5, the top row shows the current image of the interventional device. The middle row shows estimated future images of the interventional device determined using the first neural network model, where the estimated future images show the predicted progression of the interventional device from right to left. The bottom row shows future motion vectors predict for the corresponding estimated future images indicating the future movement directions of the interventional device, output by the first neural network model. In particular, the bottom row presents an overlay of the current image over the estimated future images, and illustrates the respective further motion vectors. As shown, the future motion vectors become longer the further into the future they predict. The future motion vectors may be used to determine the appropriate adjustment (e.g., angle of rotation) for the displayed image such that the movement direction in the image aligns with the control direction of the input device.

More particularly, in the depicted example, the direction of each future motion vector in the bottom row of FIG. 5 will determine the angle at which the current image in the top row will be rotated. The length of each future motion vector indicates the number of steps in the future where the desired rotation needs to be fully achieved. The steps in the future may be determined based on time and the frame rate of the image acquisition, for example. For instance, an X-ray imaging device, such as a mobile C-arm scanner, may acquire images at a frame rate between 5 to 15 images per second. By predicting the movement directions for multiple times in the future, the system may temporally filter out noisy data and use the mean or median of the future motion vectors to select optimal parameters for the displayed image.

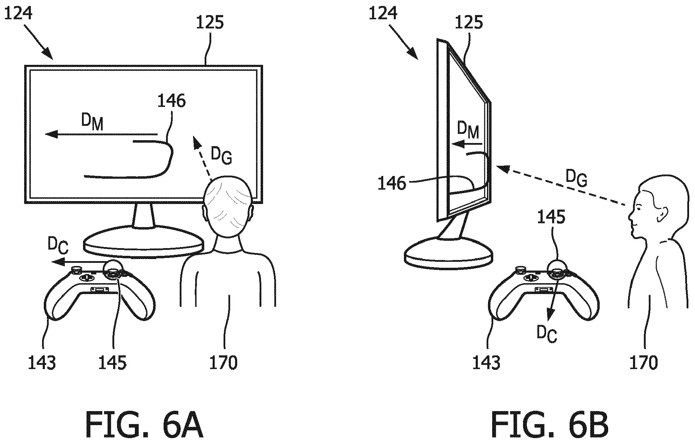

FIG. 6A shows an illustrative control console for controlling directional movement of an interventional device and an illustrative displayed image of the interventional device viewed by a user straight ahead, and FIG. 6B shows the illustrative control console for controlling directional movement of the interventional device and the illustrative displayed image of the interventional device viewed by the user at an offset angle, with matching directional orientations, according to a representative embodiment. In particular, FIGs. 6A and 6B show the situation in which the physical position and orientation of the display 124 within an exam room change in relation to a user 170. When the position and orientation of the display 124 change, the rendering of the image 125 on the display 124 will also change, disrupting previous alignment of the control direction D_{C} of the input device 145 and the movement direction D_{M} of the interventional device 146.

Referring to FIG. 6A, the display 124 is shown directly in front of the user 170 such that the user 170 views the image 125 by looking straight ahead, indicated by gaze direction D_{G}. In this configuration, it is assumed that the control direction D_{C} of the input device 145 on the control console 143 is reoriented to match the movement direction D_{M} of the interventional device 146 in the image 125. The control direction D_{C} of the input device 145 and the movement direction D_{M} of the interventional device 146 may match as originally implemented, or may have been adjusted to match as a result of reorienting one or the control direction D_{C} or the movement direction D_{M} according to the embodiments discussed above.

Referring to FIG. 6B, the display 124 is shown in a different position and orientation, offset to the right of the user 170, causing the user 170 to have to move their head to the right in order to see the image 125, indicated by the new gaze direction D_{G}. From this perspective, movement direction D_{M} appears to angle downward, and thus the control direction D_{C} of the input device 145 would no longer align with the movement direction D_{M}. Accordingly, the orientation of the input device 145 is adjusted relative to the control console 143, according to the process(es) discussed above with reference to FIG. 3, to compensate for the mismatch between the movement direction D_{M} of the interventional device 146 in the image 125, as seen with the display 124 in the different position and orientation, and the control direction D_{C} of the input device 145 on the control console 143.

In accordance with various embodiments of the present disclosure, the methods described herein may be implemented using a hardware computer system that executes software programs stored on non-transitory storage mediums. Further, in an exemplary, non-limited embodiment, implementations can include distributed processing, component/object distributed processing, and parallel processing. Virtual computer system processing may implement one or more of the methods or functionalities as described herein, and a processor described herein may be used to support a virtual processing environment.

Although estimating and visualizing trajectories of a robotically controlled interventional device on a display has been described with reference to exemplary embodiments, it is understood that the words that have been used are words of description and illustration, rather than words of limitation. Changes may be made within the purview of the appended claims, as presently stated and as amended, without departing from the scope and spirit of the embodiments. Although estimating and visualizing trajectories of a robotically controlled interventional device on a display has been described with reference to particular means, materials and embodiments, it is not intended to be limited to the particulars disclosed; rather the estimating and visualizing trajectories of a robotically controlled interventional device on a display extends to all functionally equivalent structures, methods, and uses such as are within the scope of the appended claims.

The illustrations of the embodiments described herein are intended to provide a general understanding of the structure of the various embodiments. The illustrations are not intended to serve as a complete description of all of the elements and features of the disclosure described herein. Many other embodiments may be apparent to those of skill in the art upon reviewing the disclosure. Other embodiments may be utilized and derived from the disclosure, such that structural and logical substitutions and changes may be made without departing from the scope of the disclosure. Additionally, the illustrations are merely representational and may not be drawn to scale. Certain proportions within the illustrations may be exaggerated, while other proportions may be minimized. Accordingly, the disclosure and the figures are to be regarded as illustrative rather than restrictive.

One or more embodiments of the disclosure may be referred to herein, individually and/or collectively, by the term "invention" merely for convenience and without intending to voluntarily limit the scope of this application to any particular invention or inventive concept. Moreover, although specific embodiments have been illustrated and described herein, it should be appreciated that any subsequent arrangement designed to achieve the same or similar purpose may be substituted for the specific embodiments shown. This disclosure is intended to cover any and all subsequent adaptations or variations of various embodiments. Combinations of the above embodiments, and other embodiments not specifically described herein, will be apparent to those of skill in the art upon reviewing the description.

The Abstract of the Disclosure is provided to comply with 37 C.F.R. §1.72(b) and is submitted with the understanding that it will not be used to interpret or limit the scope or meaning of the claims. In addition, in the foregoing Detailed Description, various features may be grouped together or described in a single embodiment for the purpose of streamlining the disclosure. This disclosure is not to be interpreted as reflecting an intention that the claimed embodiments require more features than are expressly recited in each claim. Rather, as the following claims reflect, inventive subject matter may be directed to less than all of the features of any of the disclosed embodiments. Thus, the following claims are incorporated into the Detailed Description, with each claim standing on its own as defining separately claimed subject matter.

The preceding description of the disclosed embodiments is provided to enable any person skilled in the art to practice the concepts described in the present disclosure. As such, the above disclosed subject matter is to be considered illustrative, and not restrictive, and the appended claims are intended to cover all such modifications, enhancements, and other embodiments which fall within the true spirit and scope of the present disclosure. Thus, to the maximum extent allowed by law, the scope of the present disclosure is to be determined by the broadest permissible interpretation of the following claims and their equivalents and shall not be restricted or limited by the foregoing detailed description.

## Claims

1. A system for displaying and controlling the progress of an interventional device configured for insertion into an anatomical structure of a subject, the system comprising:
at least one processor adapted to be coupled to (i) a display and to (ii) a user interface to provide control inputs for controlling movements of the interventional device; and
a non-transitory memory for storing instructions that, when executed by the at least one processor, cause the at least one processor to:
read a determinate coordinate system associated with the user interface, stored in said memory;
receive current image data of a current image of the interventional device in the anatomical structure displayed or to be displayed on a display, the current image showing a current position of the interventional device;
receive a control input from the user interface for controlling a movement of the interventional device from the current position, the control input being representative of a control direction in said determinate coordinate system of the user interface;
estimate from at least the current image data a movement direction of the interventional device based on the control input;
estimate a mismatch between the movement direction of the interventional device and the control direction;
determine a change of orientation of the current image displayed or to be displayed or a change of orientation of the coordinate system of the user interface to align the movement direction of the interventional device in the image and the control direction of the user interface; and
implement the change of orientation.

2. The system of claim 1, wherein determining a change of orientation of the current image displayed or to be displayed comprises rotating the current image displayed or to be displayed until the movement direction of the interventional device displayed or to be displayed aligns with the control direction.

3. The system of claim 1 or 2, wherein determining a change of orientation of the coordinate system of the user interface comprises controlling the alteration of functionality of the user interface such that a control input corresponding to the control direction matches the movement direction of the interventional device displayed or to be displayed.

4. The system of any of preceding claims, wherein estimating the movement direction of the interventional device comprises:
inferring a shape of the interventional device and surrounding anatomy of the anatomical structure from the current image and a plurality of stored recent past images; and
estimating the movement direction of the interventional device based on the shape of the interventional device and the surrounding anatomy of the anatomical structure.

5. The system of any of preceding claims, wherein estimating the movement direction of the interventional device comprises:
establishing a motion vector for the interventional device using a plurality of stored recent past images and corresponding control inputs, wherein the motion vector represents a direction and a magnitude of displacement of the interventional device moving through the anatomical structure shown in the plurality of recent past images;
predicting future motion vectors in corresponding next images indicative of a future direction of the movement of the interventional device using a first neural network model, wherein a length of the future motion vector indicates a number of future frames of the next images are needed for the movement of the interventional device to be fully realized; and
estimating the movement direction of the interventional device based on the predicted future motion vectors.

6. The system of claim 5, further comprising at least one processor and a non-transitory memory for storing instructions that, when executed by this at least one processor, cause this at least one processor to:
initially train the first neural network model using motion vectors and corresponding control inputs associated with a plurality of training images, wherein the first neural network model includes recurrent convolutional layers or transformer architectures.

7. The system of claim 5, further comprising at least one processor and a non-transitory memory for storing instructions that, when executed by this at least one processor, cause this at least one processor to:
initially train a second neural network model for estimating the mismatch between the movement direction of the interventional device and the control direction of the input device of the control console using the current image, the plurality of recent past images and corresponding control inputs, and the estimated movement direction of the interventional device.

8. The system of claim 7, wherein each of the first neural network model and the second neural network model is supervised or self-supervised.

9. The system of claim 7, wherein each of the first neural network model and the second neural network model is unsupervised.

10. The system of any of the preceding claims, further comprising (i) said user interface for controlling movements of the interventional device based on control inputs, associated with said stored determinate coordinate system, coupled to the at least one processor, and/or (ii) said display configured to display images of the interventional device in the anatomical structure of the subject, coupled to the at least one processor.

11. The system of claim 10, wherein the user interface comprises a control console comprising an input device operable by a user for controlling movement of the interventional device, the input device being optionally a joy stick or a thumb stick.

12. The system of any of the preceding claims, wherein said instructions, when executed by the at least one processor, cause the at least one processor to further:
determine said control direction of the input device relative to the control console based on the control input.

13. The system of any of preceding claims, further comprising:
an imaging system configured to acquire the current image of the anatomical structure, and a robot controller configured to enable control of the robot in accordance with the control input provided through the user interface.

14. A method of displaying and controlling the progress of an interventional device configured for insertion into an anatomical structure of a subject, the method comprising:
receiving a current image data of the interventional device in the anatomical structure of the subject, the current image being displayed or to be displayed on a display and showing a current position of the interventional device;
receiving a control input from a user interface for controlling a movement of the interventional device from the current position, the control input being representative of a control direction in a registered determinate coordinate system of the user interface;
estimating from at least the current image data a movement direction of the interventional device based on the control input;
estimating a mismatch between the movement direction of the interventional device and the control direction;
determining a change of orientation of the current image or a change of orientation of the coordinate system of the user interface to align the movement direction of the interventional device and the control direction of the user interface; and
implementing the change of orientation.

15. A non-transitory computer readable medium storing instructions, for displaying and controlling the progress of an interventional device configured for insertion into an anatomical structure of a subject, that when executed by at least one processor adapted to be coupled to (i) a display and to (ii) a user interface to provide control inputs for controlling movements of the interventional device, cause the at least one processor to:
read a determinate coordinate system associated with the user interface, stored in said memory;
receive a current image data of the interventional device in the anatomical structure displayed or to be displayed on a display, the current image showing a current position of the interventional device;
receive a control input from the user interface for controlling a movement of the interventional device from the current position, the control input being representative of a control direction in said determinate coordinate system of the user interface;
estimate from at least the current image data a movement direction of the interventional device based on the control input;
estimate a mismatch between the movement direction of the interventional device and the control direction;
determine a change of orientation of the current image or a change of orientation of the coordinate system of the user interface to align the movement direction of the interventional device in the image and the control direction of the user interface; and
implement the change of orientation.
